# EUROPEAN PATENT SPECIFICATION

(11) **EP 3 614 834 B1**
(45) Date of publication and mention of the grant of the patent: **24.07.2024**
(21) Application number: 17907840.7
(22) Date of filing: 28.04.2017
(51) Int. Cl.: A01K 13/00, A01K 1/00, A61D 7/04, A61G 10/02, A61N 5/06, B05B 7/14, A61K 9/00

(54) **TREATMENT SPACE FOR ANIMALS**
BEHANDLUNGSRAUM FÜR TIERE
ESPACE DE TRAITEMENT POUR ANIMAUX

(43) Date of publication of application: 04.03.2020
(73) Proprietor: Ukkony Oy, 37500 Lempäälä (FI)
(72) Inventor: PYYKKÖ, Jyrki, 37500 Lempäälä (FI)
(74) Representative: Kangasmäki, Reijo Holger
(86) International application number: PCT/FI2017/050320
(87) International publication number: WO 2018/197740

(56) References cited:
- EP-A1- 2 457 559
- EP-A1- 2 457 559
- EP-A1- 2 929 858
- EP-A1- 2 929 858
- WO-A1-2016/053139
- WO-A1-2016/053139
- CN-A- 101 871 269
- CN-A- 101 871 269
- DE-A1- 10 135 340
- DE-A1- 10 135 340
- DE-U1- 202015 100 684
- DE-U1- 202015 100 684
- FI-B- 126 889
- FI-B- 126 889
- No further relevant documents disclosed

## Description

The invention is a treatment space for animals according to the independent claim directed thereto, wherein the treatment space relates to so called salt room therapy for animals, particularly such as e.g. horses . Preferential embodiments are recited in the dependent claims.

In application publication AU2013245453 A1 is presented a treatment space for animals developed for the above purpose, which has a therapy room for salt therapy of one horse or for simultaneous therapy of several horses at the same time, being fastened to a so called constraint box. A salt therapy apparatus for the salt room therapy to be performed in the therapy room by inhalation in the treatment space for animals includes a salt feeding arrangement for feeding of salt dust into the therapy room e.g. by means of a so called salt room generator, an airflow arrangement for circulating air in the therapy room by means of a blower arrangement, and a control apparatus with a user interface like a control panel for adjusting/ maintaining circumstances in the therapy room as desired by means of a data processing unit.

In the solution above, the indoor circumstances of the treatment space for animals are impossible to control sufficiently, because particularly circulation of the air containing salt dust has been carried out incompletely, wherein among other things e.g. a solution for removal of salt dust containing air during a salt therapy session is suggested to take place through a door being left open. This is why, with the solution according to the publication in question it is not possible to carry out a treatment space for animals with an adequately controlled indoor climate, which is why functioning with adequate reliability of the measures for controlling the therapy room's temperature and humidity suggested to be utilized in the solution in question remains unclear.

On the other hand, though the salt room therapy as such has been found a very efficient and safe therapy method in practice particularly in treatment e.g. of horses, with it alone, however, it is not in every case possible to achieve a desired effect in therapy for example when the animals to be treated have more difficult skin or respiratory inflammations, in which case the animals need to be treated furthermore with medication, such as antibiotics etc.

Publication WO 2016/053139 discloses an individual infrared salt sauna or a training apparatus with salted air circulation for human purposes, and forms the basis for the two-part form of claim 1.

This solution comprises a closed housing, inside of which there exist infrared radiators or lamps, salt plates and thermal fans. The salt plates are placed in path of the thermal air stream so that the air stream gets enriched with negative ions.

It is the aim of the treatment space for animals according to the present invention to achieve a decisive improvement in the problems described above and thus to achieve a decisive improvement particularly with a view to the salt room therapy for animals, so that for example unnecessary medications of animals can be avoided. The treatment space for animals according to the invention is primarily characterized by what has been presented in the characterizing part of the independent claim directed thereto.

As the most important advantages of the treatment space for animals according to the invention may be mentioned simplicity of it's functioning as well as of the stock of equipment related thereto and efficiency of the comprehensive therapy being produced by the same, which is achieved by combining with the salt room therapy a simultaneous heat radiation therapy of the animal to be treated. Thanks to the heat radiation therapy the metabolism and circulatory e.g. of a horse to be treated, accelerate when the heart rate thereof rises, wherein the horse's inspiratory gets stronger. Thanks to the above, the air containing salt dust, being brought to the therapy room e.g. by a customary salt room generator, is able to carry salt dust more efficiently to the horse's cells and respiratory tracts. With electric infrared radiators, being used as an advantageous embodiment of the salt room therapy, thanks to the heat therapy being accurately targeted e.g. at a horse to be treated, the salt dust is able to penetrate deeper into the cells, in which case also skin symptoms of the horse can be healed thanks to the deadly effect on the bacteria of salt. Thus, e.g. in treatment of wounds, the natural healing process of the horse can be improved along with formation of epithelial tissues and collagen.

Surface temperature of the dust/water tight infrared radiators, being exploited as an advantageous embodiment of the invention, is steplessly adjustable optimally for any given therapy situation e.g. by using an ordinary control panel or, when needed, in addition to the same or instead of the same through threadless remote control. The treatment space for animals can be furthermore implemented according to the invention as a structure entirety that is essentially isolated by its indoor climate with respect to its surroundings, wherein it has a totally controllable indoor climate thanks to a mechanical ventilation, or when needed, also thanks to a cooling air conditioning, particularly for removing of salty air in a controlled and computer assisted manner controlled by a monitoring arrangement, in order to adjust/maintain the circumstances in the therapy room, such as its salinity, humidity, pressure and/or temperature, as desired.

Use of energy and surface temperatures of the heat radiators in the treatment space for animals according to the invention are as advantageous embodiments adjustable optimally from the point of view of the animal to be treated by carrying out the radiant heater apparatus with an infrared radiant heat arrangement that produces heat radiation essentially in horizontal and vertical directions, wherein distance of the heat radiators with respect to the animal to be treated is adjustable. In this way it is possible to optimize the surface temperature of the heat radiators, while at the same time unnecessary heating of the therapy room's indoor air is being minimized.

Other advantageous embodiments of the treatment space for animals according to the invention are presented in the dependent claims directed thereto.

In the following description the invention is illustrated in detail with reference to the accompanying drawings, in which:
In figure 1
   is shown with a perspective view a general principle of the treatment space for animals according to the invention,
In figure 2
   is shown with a perspective view by way of illustration a horizontal part entirety in a radiant heater apparatus of the treatment space for animals according to the invention,
In figure 3
   is shown furthermore with a perspective view by way of illustration a vertical part entirety in a radiant heater apparatus of the treatment space for animals according to the invention, and
In figure 4
   is shown with a perspective view an advantegous prefabricated assembly of the treatment space for animals according to the invention.

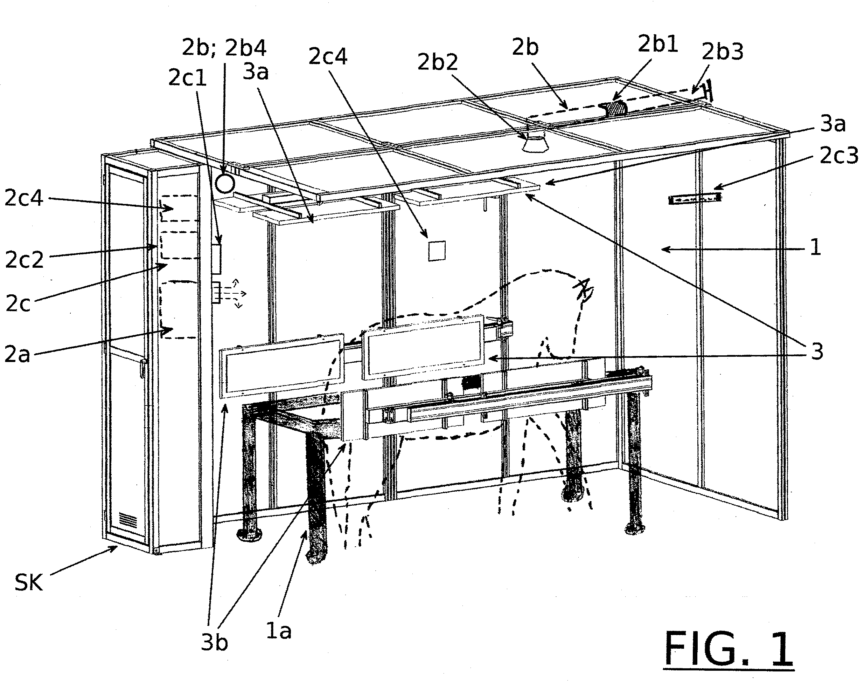

The invention relates to a treatment space for animals, which comprises a therapy room 1 for therapy treatment of one or several animals simultaneously, such as a horse to be fastened to a constraint box, and a salt therapy apparatus for salt room therapy to be performed in the therapy room by inhalation, which salt therapy apparatus includes a salt feeding arrangement 2a for feeding of salt dust into the therapy room, such as a salt room generator, an airflow arrangement 2b for circulating air in the therapy room, such as a blower arrangement 2b1, and a control apparatus 2c with a user interface 2c1, like a control panel, for adjusting/ maintaining circumstances in the therapy room as desired by means of a data processing unit 2c2. Particularly in order to make the salt room therapy more efficient by exploiting heat, the treatment space for animals comprises e.g with reference to the general principle shown in figure 1 a radiant heater apparatus 3, being arranged in connection with the therapy room 1.

As an advantageous embodiment of the treatment space for animals according to the invention, the radiant heater apparatus 3 comprises one or more electric heat radiators 3a, 3b, such as an infrared radiant heater arrangement or like. There are at present available on the market infrared radiators especially suitable for the purpose explained above particularly by dust and water tightness thereof with product name IP67 (Rauduspuu Oy), the surface temperature of which is adjustable according to need between 0-160 °C.

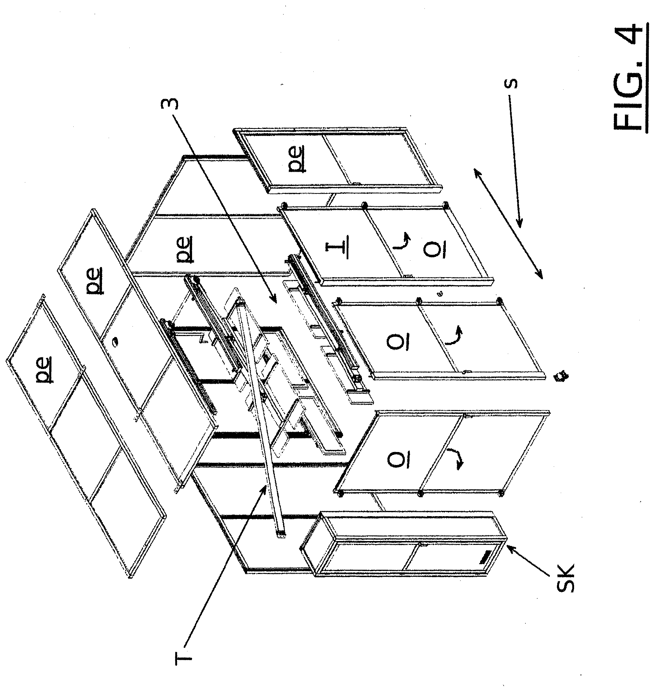

As a furthermore advantageous embodiment of the treatment space for animals according to the invention, the radiant heater apparatus 3 comprises on the principle as shown in figures 1 and 4 an infrared radiant heater arrangement 3a, 3b producing essentially horizontal and vertical heat radiation.

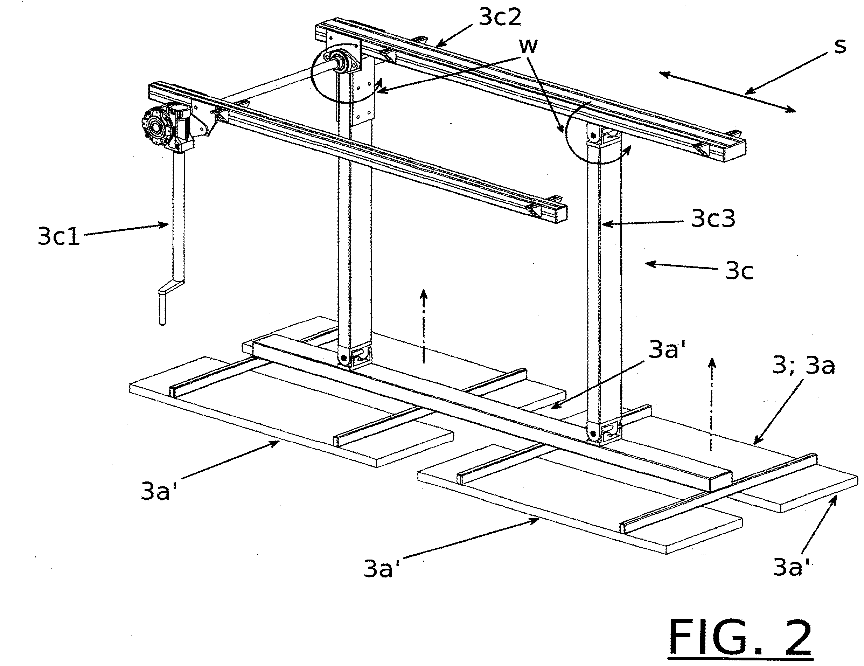

In this context, as a furthermore advantageous embodiment, the radiant heater apparatus 3 comprises e.g. according to figure 2 one or more heat radiators 3a, being arranged essentially horizontally above the therapy room 1.

In this context, as a furthermore advantageous embodiment, the radiant heater apparatus 3 comprises at least two heat radiators 3b, e.g. according to figure 2, being arranged essentially in a vertical plane opposite to each other at the sides of the therapy room 1.

Particularly the radiant heater apparatuses 3 being presented in figures 1-4 are put together by the horizontal heat radiators 3a with four heat radiator elements 3a' and respectively by the vertical heat radiators 3b with two radiator elements 3b'.

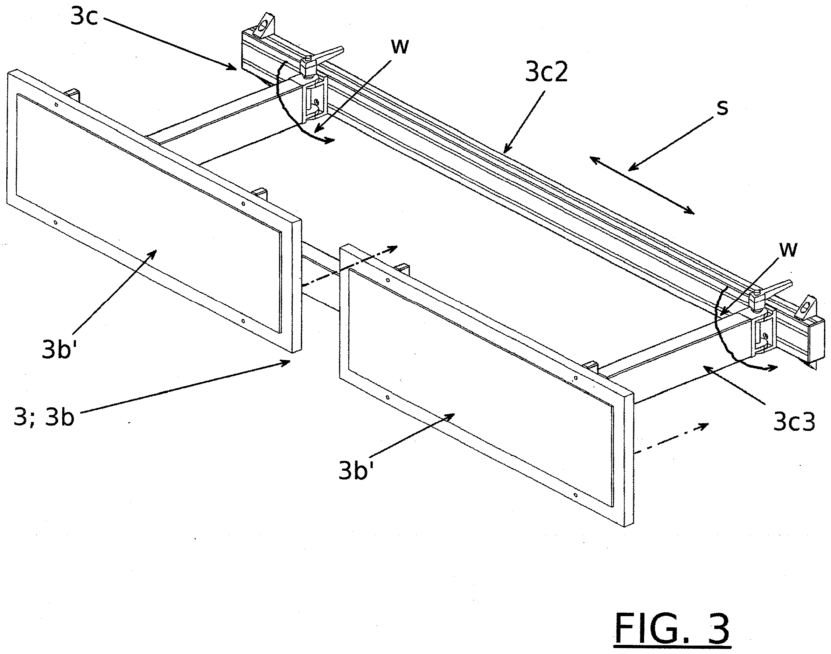

As a furthermore advantageous embodiment of the treatment space for animals according to the invention, particularly with reference to figures 2 and 3, the radiant heater apparatus 3 is provided with an adjustment apparatus 3c for adjusting a distance of the one or more heat radiators 3a, 3b in relation to an animal to be treated. In the implementation according to figure 2, the vertical adjustment of the horizontal heat radiator 3a is carried out mechanically by fastening arms 3c3 of the heat element to be tilted manually with a so called crank arm 3c1, wherein the fastening arms are arranged furthermore movable in the longitudinal direction s of the therapy room by means of a glide arrangement 3c2 for adjusting the place of the heat radiator 3a. In figure 3 is shown an adjustment of the vertical heat radiator 3b to be carried manually on the corresponding principle.

As a furthermore advantageous embodiment of the treatment space for animals according to the invention, the adjustment apparatus 3c of the radiant heater apparatus 3 is arranged to be operated by means of auxiliary power by using electrically, pressure medium and/or combustion engine operated actuators (which are not, however, presented in greater detail in the drawings). In this context, as is the case also with exploiting manual adjustment, it is naturally possible to carry out the length of the fastening arms 3c3 of the heat radiators 3a, 3b adjustable on telescope principle.

As a furthermore advantageous embodiment of the treatment space for animals according to the invention, the treatment space comprises, particularly with reference to figure 4, a structure entirety, being put together from floor, wall and roof pe as well as openable door structures and being provided advantageously in practise with window surfaces I enabling visibility to the therapy room from outside the treatment space for animals.

According to the invention, the treatment space, being assembled, when needed, with thermally insulated structures, is arranged as a structure entirety isolated by its indoor climate from its surroundings, wherein it has feed air 2b;2b4 and exhaust air arrangements 2b;2b2 for removing salty air of the therapy room 1 by means of a data processing unit 2c2, such as one or more computers, programmable logics, microprocessors and/or like, and a control unit 3c4 of the radiant heater apparatus 3, being coupled with the data processing unit, and controlled by a monitoring arrangement 2c3 in connection with the therapy room, such as by one or more salinity detectors.

As a furthermore advantageous embodiment of the treatment space for animals according to the invention, its control apparatus 2c comprises a monitoring arrangement 2c4 also for adjusting/maintaining other circumstances of the therapy room 1 as desired, such as its humidity, pressure, temperature and/or the like. By means of a monitoring arrangement being carried out in this way, it is furthermore possible to optimize the indoor climate of the treatment space for animals in every respect by utilizing, when needed, cooling, humidification or the like auxiliary functionings. This way it is possible to carry out a controlled indoor climate of the treatment space for animals on a principle, corresponding to so called mechanical ventilation or air conditioning, by maintaining adequate supply of fresh air in the therapy room during the salt room therapy.

As a furthermore advantageous embodiment of the treatment space for animals according to the invention, in connection therewith is arranged a post processing apparatus for recovering heat and/or salt from the salty air to be removed from therapy room 1 (which principle has not, however, been presented in greater detail in the drawings), thanks to which it is furthermore possible to optimize energy consumption of the treatment space for animals as well as minimize the amount of salt that gets drifted to the surroundings.

As a furthermore advantageous embodiment with reference to the assembly shown in figure 4, the treatment space for animals according to the invention can be carried out for example on so called module principle to be put together from separate elements as an entirety with necessary binding structures T, a control center SK and a support structure for the stock of equipment of the treatment space.

It is clear, that the invention is not limited to the presented or described embodiments above, but instead it can be varied depending on the animals to be treated at any given time and on the sizes thereof (for example dogs/cats/cows/horses).

## Claims

1. Treatment space for animals, which comprises a therapy room (1) for therapy treatment of one or several animals simultaneously, such as a horse to be fastened to a constraint box, a radiant heater apparatus (3) and a salt therapy apparatus for salt room therapy to be performed in the therapy room by inhalation, which salt therapy apparatus includes a salt feeding arrangement (2a) for feeding of salt dust into the therapy room including a salt room generator, an airflow arrangement (2b) for circulating air in the therapy room by means of a blower arrangement (2b1), and a control apparatus (2c) with a user interface (2c1) comprising a control panel, for adjusting/maintaining circumstances in the therapy room as desired by means of a data processing unit (2c2) comprising one or more computers, programmable logics and/or microprocessors, **characterized in that** the airflow arrangement (2b) within the treatment space for animals comprises a feed air arrangement (2b;2b4) and an exhaust air arrangement (2b;2b2) for removing salty air from the therapy room (1), in connection with a control unit (3c4) being coupled with the data processing unit (2c2) and controlled by a therapy room monitoring arrangement (2c3), consisting at least of one or more salinity detectors.

2. Treatment space for animals according to claim 1, **characterized in that**, the radiant heater apparatus (3) comprises one or more electric heat radiators (3a, 3b), comprising of an infrared radiant heater arrangement or like.

3. Treatment space for animals according to claim 1 or 2, **characterized in that**, the radiant heater apparatus (3) comprises an infrared radiant heater arrangement (3a, 3b) producing essentially horizontal and vertical heat radiation.

4. Treatment space for animals according to any of the preceding claims 1-3, **characterized in that**, the radiant heater apparatus (3) comprises one or more heat radiators (3a), being arranged essentially horizontally above the therapy room (1).

5. Treatment space for animals according to any of the preceding claims 1-4, **characterized in that**, the radiant heater apparatus (3) comprises at least two heat radiators (3b), being arranged essentially in a vertical plane opposite to each other at the sides of the therapy room (1).

6. Treatment space for animals according to any of the preceding claims 1-5, **characterized in that**, the radiant heater apparatus (3) is provided with an adjustment apparatus (3c) for adjusting a distance of the one or more heat radiators (3a, 3b) in relation to an animal to be treated.

7. Treatment space for animals according to claim 6, **characterized in that**, the adjustment apparatus (3c) of the radiant heater apparatus (3) is arranged to be operated by means of auxiliary power by using electrically, pressure medium and/or combustion engine operated actuators.

8. Treatment space for animals according to any of the preceding claims 1-7, wherein the treatment space comprises a structure entirety, being put together from floor, wall and roof (pe) as well as openable door structures and being preferably provided with window surfaces (I), **characterized in that**, the treatment space being assembled, when needed, with thermally insulated structures, is arranged as a structure entirety isolated by its indoor climate from its surroundings.

9. Treatment space for animals according to any of the preceding claims 1-8, **characterized in that**, its control apparatus (2c) comprises a monitoring arrangement (2c4) also for adjusting/maintaining other circumstances of the therapy room (1) as desired, such as its humidity, pressure, temperature and/or the like.

10. Treatment space for animals according to any of the preceding claims 1-8, **characterized in that**, in connection therewith is arranged a post processing apparatus for recovering heat and/or salt from the salty air to be removed from therapy room (1).

## Patentansprüche

1. Behandlungsraum für Tiere, der einen Therapieraum (1) zur gleichzeitigen therapeutischen Behandlung eines oder mehrerer Tiere, wie beispielsweise eines in einer Anbindebox anzuleinenden Pferdes, eine Heizstrahlervorrichtung (3) und eine Salztherapievorrichtung für eine im Therapieraum durch Inhalation durchzuführende Salzraumtherapie umfasst, wobei die Salztherapievorrichtung eine Salzzuführungsanordnung (2a) zum Zuführen von Salzpartikeln in den Therapieraum einschließlich eines Generators, einer Luftstromanordnung (2b) zum Umwälzen von Luft in dem Therapieraum mittels einer Gebläseanordnung (2b1) und einer Steuervorrichtung (2c) mit einer Benutzerschnittstelle (2c1) samt Bedienfeld zum Einstellen/Aufrechterhalten von in dem Therapieraum gewünschten Verhältnissen mittels einer Datenverarbeitungseinheit (2c2), die einen oder mehrere Rechner, programmierbare Bausteine und/oder Mikroprozessoren umfasst, enthält, **dadurch gekennzeichnet, dass** die Luftstromanordnung (2b) innerhalb des Behandlungsraums für Tiere eine Zuluftanordnung (2b;2b4) und eine Abluftanordnung (2b;2b2) zum Abführen von salzhaltiger Luft aus dem Behandlungsraum (1) in Verbindung mit einer mit der Datenverarbeitungseinheit (2c2) gekoppelten Steuereinheit (3c4) umfasst, die von einer Behandlungsraum-Überwachungsanordnung (2c3) gesteuert wird, die mindestens aus einem oder mehreren Salzgehaltsdetektoren besteht.

2. Behandlungsraum für Tiere nach Anspruch 1, **dadurch gekennzeichnet, dass** die Heizstrahlervorrichtung (3) einen oder mehrere elektrische Heizstrahler (3a, 3b) umfasst, die aus einer Infrarot-Heizstrahleranordnung oder dergleichen bestehen.

3. Behandlungsraum für Tiere nach Anspruch 1 oder 2, **dadurch gekennzeichnet, dass** die Heizstrahlervorrichtung (3) eine Infrarot-Heizstrahleranordnung (3a, 3b) umfasst, die im Wesentlichen eine horizontale und vertikale Wärmestrahlung erzeugt.

4. Behandlungsraum für Tiere nach einem der vorstehenden Ansprüche 1 bis 3, **dadurch gekennzeichnet, dass** die Heizstrahlervorrichtung (3) einen oder mehrere Heizstrahler (3a) umfasst, der bzw. die im Wesentlichen über dem Therapieraum (1) angeordnet sind.

5. Behandlungsraum für Tiere nach einem der vorhergehenden Ansprüche 1 bis 4, **dadurch gekennzeichnet, dass** die Heizstrahlervorrichtung (3) mindestens zwei Wärmestrahler (3b) umfasst, die im Wesentlichen in einer vertikalen Ebene einander gegenüberliegend an den Seiten des Therapieraums (1) angeordnet sind.

6. Behandlungsraum für Tiere nach einem der vorhergehenden Ansprüche 1 bis 5, **dadurch gekennzeichnet, dass** die Heizstrahlervorrichtung (3) mit einer Einstellvorrichtung (3c) zum Einstellen eines Abstandes eines oder mehrerer Heizstrahler (3a, 3b) in Bezug auf ein zu behandelndes Tier versehen ist.

7. Behandlungsraum für Tiere nach Anspruch 6, **dadurch gekennzeichnet, dass** die Einstellvorrichtung (3c) des Heizstrahlers (3) mittels Hilfsenergie unter Verwendung von elektrischen, druckmittelbetriebenen und/oder verbrennungsmotorischen Stellgliedern betätigt werden kann.

8. Behandlungsraum für Tiere nach einem der vorhergehenden Ansprüche 1 bis 7, wobei der Behandlungsraum ein aus Boden, Wand und Dach (pe) sowie zu öffnenden Türkonstruktionen zusammengesetztes bauliches Ganzes umfasst und vorzugsweise mit Fensterflächen (I) versehen ist, **dadurch gekennzeichnet, dass** der Behandlungsraum, der bei Bedarf aus wärmegedämmten Konstruktionen zusammengesetzt ist, als ein bauliches Ganzes angeordnet ist, das durch sein Raumklima von seiner Umgebung isoliert ist.

9. Behandlungsraum für Tiere nach einem der vorhergehenden Ansprüche 1 bis 8, **dadurch gekennzeichnet, dass** seine Steuervorrichtung (2c) eine Überwachungseinrichtung (2c4) auch zur Einstellung/Aufrechterhaltung anderer Verhältnisses des Therapieraums (1), wie beispielsweise Feuchtigkeitsgehalt, Druck, Temperatur und/oder dergleichen, umfasst.

10. Behandlungsraum für Tiere nach einem der vorhergehenden Ansprüche 1 bis 8, **dadurch gekennzeichnet, dass** in Verbindung damit eine Nachbehandlungsvorrichtung zur Rückgewinnung von Wärme und/oder Salz aus der aus dem Behandlungsraum (1) abzuziehenden salzhaltigen Luft angeordnet ist.

## Revendications

1. Espace de traitement pour animaux, comprenant une salle de thérapie (1) pour le traitement thérapeutique d'un ou plusieurs animaux simultanément, tels qu'un cheval devant être attaché dans un box de contention, un appareil de chauffage radiant (3) et un dispositif de thérapie par le sel pour salle de thérapie par le sel à exécuter dans la salle de thérapie par inhalation, lequel dispositif de thérapie par le sel comprend un dispositif d'alimentation en sel (2a) pour l'injection de poussière de sel dans la salle de thérapie avec générateur de chambre de sel, un dispositif de flux d'air (2b) pour la circulation de l'air dans la salle de thérapie au moyen d'un dispositif souffleur 2b1), et un appareil de commande (2c) avec une interface utilisateur (2c1) comprenant un panneau de commande, pour ajuster/maintenir les conditions souhaitées dans la salle de thérapie au moyen d'une unité de traitement de données (2c2) comprenant un ou plusieurs ordinateurs, logiques programmables et/ou microprocesseurs, **caractérisé par le fait que** le dispositif de flux d'air (2b) dans l'espace de traitement pour animaux comprend un dispositif d'alimentation en air (2b;2b4) et un dispositif d'évacuation d'air (2b;2b2) pour éliminer l'air salé de la salle de thérapie (1), en lien avec une unité de commande (3c4) accouplée à l'unité de traitement de données (2c2) et commandée par un dispositif de surveillance de la salle de thérapie (2c3), composé au moins d'un ou plusieurs détecteurs de salinité.

2. Espace de traitement pour animaux décrit dans la revendication 1, **caractérisé par le fait que** l'appareil de chauffage radiant (3) comprend un ou plusieurs radiateurs thermiques électriques (3a, 3b), composé d'un dispositif de chauffage radiant infrarouge ou similaire.

3. Espace de traitement pour animaux décrit dans la revendication 1 ou 2, **caractérisé par le fait que** l'appareil de chauffage radiant (3) comprend un dispositif de chauffage radiant infrarouge (3a, 3b) produisant un rayonnement thermique essentiellement horizontal et vertical.

4. Espace de traitement pour animaux décrit dans une quelconque des revendications précédentes 1 à 3, **caractérisé par le fait que** l'appareil de chauffage radiant (3) comprend un ou plusieurs radiateurs thermiques (3a), disposés essentiellement horizontalement au-dessus de la salle de thérapie (1).

5. Espace de traitement pour animaux décrit dans une quelconque des revendications précédentes 1 à 4, **caractérisé par le fait que** l'appareil de chauffage radiant (3) comprend au moins deux radiateurs thermiques (3b), disposés essentiellement sur un plan vertical et opposés l'un à l'autre sur les côtés de la salle de thérapie (1).

6. Espace de traitement pour animaux décrit dans une quelconque des revendications précédentes 1 à 5, **caractérisé par le fait que** l'appareil de chauffage radiant (3) est pourvu d'un appareil de réglage (3c) pour ajuster la distance du ou des radiateurs thermiques (3a, 3b) par rapport à l'animal à traiter.

7. Espace de traitement pour animaux décrit dans la revendication 6, **caractérisé par le fait que** l'appareil de réglage (3c) de l'appareil de chauffage radiant (3) est disposé de sorte à fonctionner au moyen d'énergie auxiliaire à l'aide d'actionneurs à commande électrique, par fluide sous pression et/ou moteur à combustion.

8. Espace de traitement pour animaux décrit dans une quelconque des revendications précédentes 1 à 7, dans lequel l'espace de traitement comprend une structure intégrale, assemblée depuis le sol, les murs et le toit (pe) et pouvant être ouvert par des structures et de préférence équipé de surfaces de fenêtre (I), **caractérisé par le fait que** l'espace de traitement assemblé, si nécessaire, avec des structures thermiquement isolées, est disposé comme une structure intégralement isolée de son environnement par son climat intérieur.

9. Espace de traitement pour animaux décrit dans une quelconque des revendications précédentes 1 à 8, **caractérisé par le fait que** son appareil de commande (2c) comprend un dispositif de surveillance (2c4) également pour ajuster/maintenir les autres conditions de la salle de thérapie (1) au niveau souhaité en matière d'humidité, de pression, de température et/ou autres.

10. Espace de traitement pour animaux décrit dans une quelconque des revendications précédentes 1 à 8, **caractérisé par le fait qu'**en lien avec cet espace est disposé un appareil de post-traitement pour récupérer la chaleur et/ou le sel de l'air salé à extraire de la salle de thérapie (1).
